# EUROPEAN PATENT SPECIFICATION

(11) **EP 2 019 464 B1**
(45) Date of publication and mention of the grant of the patent: **08.04.2015**
(21) Application number: 07743012.2
(22) Date of filing: 09.05.2007
(51) Int. Cl.: H01T 19/00, A61L 9/18, B01D 53/38, B01J 19/08, B05B 5/025, B05B 5/08, H05H 1/24, B03C 3/38

(54) **DISCHARGE DEVICE AND AIR PURIFYING DEVICE**
ENTLADUNGSEINRICHTUNG UND LUFTREINIGUNGSEINRICHTUNG
MÉCANISME D'ÉMISSION ET MÉCANISME DE PURIFICATION D'AIR

(30) Priority: 19.05.2006 JP 2006140104; 15.06.2006 JP 2006166237
(43) Date of publication of application: 28.01.2009
(73) Proprietor: Daikin Industries, Ltd., Osaka-shi, Osaka 530-8323 (JP)
(72) Inventor: MOTEGI, Kanji, Osaka 591-8511 (JP); ODO, Tsunahiro, Osaka 591-8511 (JP); TANAKA, Toshio, Osaka 591-8511 (JP); AKIYAMA, Ryuji, Osaka 591-8511 (JP)
(74) Representative: Goddar, Heinz J.
(86) International application number: PCT/JP2007/059577
(87) International publication number: WO 2007/135860

(56) References cited:
- WO-A1-96/06706
- JP-A- 60 019 022
- JP-A- 2003 053 129
- JP-A- 2006 102 831

## Description

### TECHNICAL FIELD

The present invention relates to the field of electric discharge devices for the generation of a low temperature plasma by means of electrical discharge, and to the field of air purification devices for the purification of air with the aid of a low pressure plasma generated by such an electric discharge device.

### BACKGROUND ART

One such electric discharge device is shown in JP-A-2003-038932 which is referred to hereinafter as the "patent document". This prior-art electric discharge device is configured such that streamer discharge is generated towards a sheet-shaped counter electrode from a needle-shaped discharge electrode. In this electric discharge device, with the generation of a streamer discharge, a low temperature plasma is produced to cause the generation of active species (e.g., electrons, various excited molecules et cetera). And, when air to be treated is flowing through the low temperature plasma, odorous and/or noxious components contained in the air to be treated are decomposed by the active species into CO₂ and H₂O. Consequently, the air to be treated is deodorized. In addition, the device according to the patent document employs also a catalyst to enhance the activation of active species for the acceleration of treatment.

Furthermore, WO-96/06706 describes a reactor and method for the treatment of matter by plasma action/using a plurality of electrode structures for the treatment of particulate matter. Moreover, DE 10 2005 044 603 A1 describes an electric discharge device and an air purification device according to the preambles of the independent claims.

### DISCLOSURE OF THE INVENTION

### PROBLEMS THAT THE INVENTION INTENDS TO OVERCOME

However, the generation of streamer discharges in the electric discharge device of the patent document requires careful settings for the tip shape of the discharge electrode, the interelectrode distance between the discharge electrode and the counter electrode, and the applied voltage according to the interelectrode distance. However, in spite of such careful settings, there are possible problems. More specifically, if the interelectrode distance varies due to the wear/damage of the discharge electrode associated with electric discharge or if pollutants adhere to the discharge electrode, this may result in the occurrence of an electric spark instead of the generation of a streamer discharge or result in the generation of a weak electric discharge such as a glow corona discharge. As described above, it has been extremely difficult for the conventional electric discharge device to stably maintain the state of electric discharge (especially, streamer discharge) at a desired level.

The invention was made in view of the above problems. Accordingly, an object of the invention is to stabilize the state of electric discharge in an electric discharge device. Another object is to stabilize the performance of an air purification device by the application of such an electric discharge device thereto.

### MEANS FOR OVERCOMING THE PROBLEMS

The present invention is defined by the subject matter of the independent claims. Preferred embodiments can be derived from the dependent claims and the description. According to claim 1, which corresponds to a first aspect of the description, there is provided an electric discharge device **(20),** which includes a discharge electrode **(21),** a counter electrode **(22),** and an electric power supply **(23)** for providing an electric potential difference between both the electrodes **(21, 22),** for the generation of an electric discharge towards the counter electrode **(22)** from the discharge electrode **(21).**

The electric discharge device **(20)** of the first aspect is provided with a granular particulate ejector **(24)** configured to eject granular particulates **(22)**, and the counter electrode **(22)** is composed of the granular particulates **(22)** ejected towards the discharge electrode **(21)** from the granular particulate ejector **(24)**. It should be noted that the "granular particulate **(22)**", as termed herein, is not necessarily limited only to the "granular particulate **(22)**", in other words it may be in the form of a "powder" or in the form of a "liquid droplet **(22)**". In addition, the discharge electrode **(21)** may be formed by, for example, a needle-shaped electrode **(21a)**.

Since, in the invention, the counter electrode **(22)** is in the form of granular particulates, this creates a space between the granular particulates. In the absence of such a space, there may be a continuous series of electric discharges, resulting in causing electric sparking. In the first aspect, however, electric sparking is unlikely to be produced, and the state of electric discharge becomes stabilized.

In a second aspect according to the first aspect of the description, it is characterized in that the discharge electrode **(21)** is formed by a plurality of needle-shaped electrodes **(21a)**.

Since, in the second aspect of the description, a plurality of needle-shaped electrodes **(21a)** are provided as the discharge electrode **(21)**, this makes it possible that, even when the state of electric discharge from some needle-shaped electrodes **(21a)** becomes unsteady, the stable state of electric discharge is continuously maintained in the rest of the needle-shaped electrodes **(21a)**.

In a third aspect according to the first or second aspect of the description, it is characterized in that the mutual relationship in position between the discharge electrode **(21)** and the granular particulate ejector **(24)** is determined so that, depending on the electric potential difference between the discharge electrode **(21)** and the counter electrode **(22)**, there is generated between the discharge electrode **(21)** and the granular particulates **(22)** a streamer discharge.

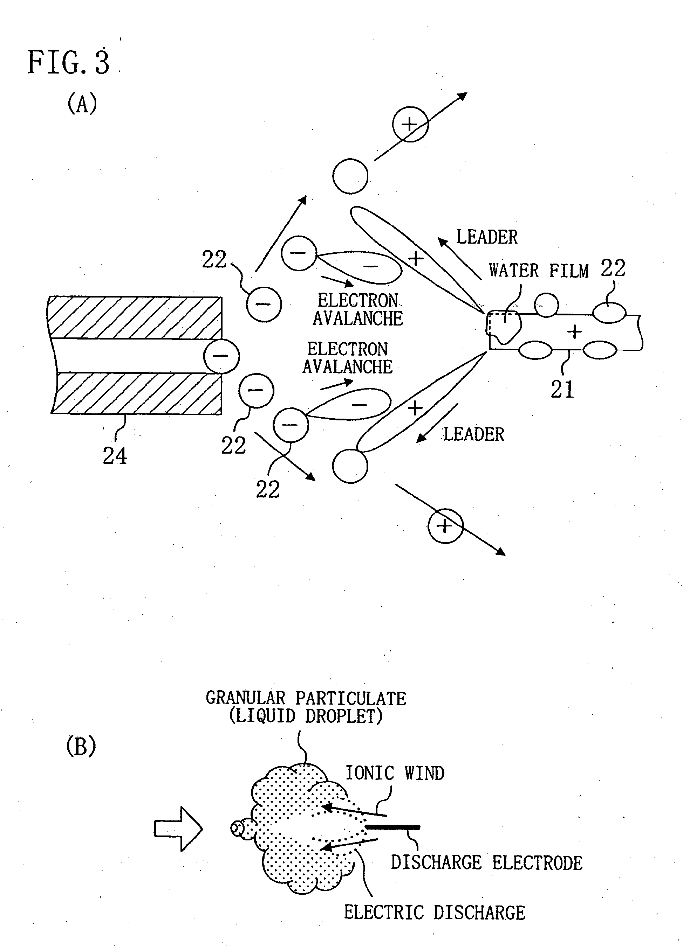

In the third aspect of the description, for example, the discharge electrode **(21)** serves as a positive pole and the granular particulate ejector **(24)** serves as a negative pole and these electrodes **(21, 24)** are arranged face to face with each other (see Figure **3(A)**). As a result of such arrangement, the granular particulate **(22)** jumps out from the granular particulate ejector **(24)** with negative electric charges. And an electron avalanche takes place from the negatively charged granular particulate **(22)** towards the positively charged discharge electrode **(21),** as a result of which the granular particulate **(22)** losses its negative electric charges. Meanwhile, a minute electric arc called a "leader" of positive electric charge advances from the discharge electrode **(21)** and reaches the granular particulate **(22)**. Here, if the positively charged granular particulate **(22)** stands still or is traveling in the direction of the granular particulate ejector **(24)**, this produces the possibility that, with the movement of the granular particulate **(22)**, the leader may advance further to change to an electric spark. However, the granular particulate **(22)** travels in a direction moving away from the granular particulate ejector **(24)** and, besides, a space is existing between the granular particulates **(22)**. Therefore, the leader will not advance any further, in other words, the leader is prevented from changing to an electric spark. Streamer discharge is an electric discharge that repeats a series of cycles (i.e., "electron avalanche"→"leader formation"→ "leader extinction"→"electron avalanche" and so on). By virtue of the configuration of the third aspect of the description, such a series of cycles is repeated in ideal form whereby streamer discharge is stably formed as a plasma column with luminescence.

In a fourth aspect according to any one of the first to third aspects of the description, it is characterized in that the granular particulate ejector **(24)** is formed by a liquid droplet ejector **(24)** configured to spray liquid droplets **(22)**, and that the counter electrode **(22)** is composed of the liquid droplets **(22)** ejected towards the discharge electrode **(21)** from the liquid droplet ejector **(24)**.

Since, in the fourth aspect of the description, the counter electrode **(22)** is in the form of liquid droplets **(22)**, this creates a space between the liquid droplets **(22)**. In the absence of such a space, there may be a continuous series of electric discharges, resulting in causing electric sparking. In the fourth aspect, however, electric sparking is unlikely to be produced, and the state of electric discharge (streamer discharge) becomes stabilized.

In a fifth aspect according to any one of the first to fourth aspects of the description, it is characterized in that the direction of ejection of the granular particulates from the granular particulate ejector **(24)** is substantially oriented to the discharge electrode **(21)**, and that the tip of the discharge electrode **(21)** is substantially oriented to the granular particulate ejector **(24)**.

In the fifth aspect of the description, the state of electric discharge becomes stabilized in the arrangement where the granular particulate ejector **(24)** and the discharge electrode **(21)** are situated face to face with each other. Especially, in the case where the electric discharge device **(20)** gives rise to a streamer discharge, an electron avalanche takes place when the granular particulate **(22)** is ejected from the granular particulate ejector **(24)** towards the discharge electrode **(21)**, and after the advance of a leader from the tip of the discharge electrode **(21)** toward the granular particulate, the leader will cease to exist. This is repeated, thereby causing repetition of a series of cycles (i.e., "electron avalanche"→"leader formation"→ "leader extinction"→"electron avalanche" and so on) in ideal form, whereby streamer discharge is stably formed as a plasma column with luminescence.

In a sixth aspect according to the fifth aspect of the description, it is characterized in that the shape of ejection pattern of the granular particulates **(22)** from the granular particulate ejector **(24)** is a hollow circular cone.

In the sixth aspect of the description, in the arrangement where the granular particulate ejector **(24)** and the discharge electrode **(21)** are situated face to face with each other, the granular particulates **(22)** are ejected from the granular particulate ejector **(24)** towards a hollow circular conical region around the discharge electrode **(21)**. If streamer discharge is initiated in this configuration, then the discharge region is formed into a space with a flared spread.

In a seventh aspect according to the fifth aspect of the description, it is characterized in that the shape of ejection pattern of the granular particulates **(22)** from the granular particulate ejector **(24)** is a solid circular cone.

In the seventh aspect of the description, in the arrangement where the granular particulate ejector **(24)** and the discharge electrode **(21)** are situated face to face with each other, the granular particulates **(22)** are ejected from the granular particulate ejector **(24)** towards a solid circular conical region including the discharge electrode **(21)**. If streamer discharge is initiated in this configuration, then the discharge region is formed into a column-shaped space with almost no spread.

In an eighth aspect according to any one of the first to fourth aspects of the description, it is characterized in that the tip of the discharge electrode **(21)** is substantially oriented from sideways to the direction of flow of the granular particulates **(22)** ejected almost linearly from the granular particulate ejector **(24)**.

In the eighth aspect of the description, the state of electric discharge becomes stabilized in the configuration in which the discharge electrode **(21)** is arranged lateral to the direction of flow of the granular particulates from the granular particulate ejector **(24)**. Especially, in the case where the electric discharge device **(20)** generates streamer discharges, a series of cycles (i.e., "electron avalanche"→"leader formation"→ "leader extinction"→"electron avalanche" and so on) is repeated in ideal form with respect to the flow of granular particulates **(22)** from the granular particulate ejector **(24)**, and streamer discharge is stably formed as a plasma column with luminescence.

In a ninth aspect according to any one of the first to eighth aspects of the description, it is characterized in that there is provided a granular particulate recovery container **(45)** for the recovery of the granular particulates **(22)** ejected from the granular particulate ejector **(24).**

In the ninth aspect of the description, the granular particulates **(22)** ejected from the granular particulate ejector **(24)** are collected by the granular particulate recovery container **(45).**

In a tenth aspect according to the ninth aspect of the description, it is characterized in that there is provided a granular particulate circulating mechanism **(60)** for the return of the granular particulates **(22)** recovered in the granular particulate recovery container **(45)** towards the granular particulate ejector **(24)**.

In the tenth aspect of the description, the granular particulates **(22)** recovered in the granular particulate recovery container **(45)** are returned towards the granular particulate ejector **(24)**. And these granular particulates **(22)** are again ejected from the granular particulate ejector **(24)**.

According to another aspect of the invention, which corresponds to an eleventh aspect of the description, there is provided an air purification device for the treatment of air to be treated and containing an odorous component. The air purification device of the eleventh aspect includes an electric discharge device **(20)** for the decomposition of the odorous component and the electric discharge device **(20)** is formed by any one of the electric discharge devices **(20)** as set forth in the first to tenth aspects of the description.

In the eleventh aspect of the description, electric discharge is initiated between the granular particulates **(22)** ejected from the granular particulate ejector **(24)** and the discharge electrode **(21)** and a low temperature plasma gas is generated in the discharge field. This gas contains active species such as fast electrons, ions, ozone, radicals (e.g., hydroxyl radicals and so on), and other excited molecules (e.g., excited oxygen molecules, excited nitrogen molecules, excited water molecules et cetera). And these active species render the air to be treated odorless and harmless.

In a twelfth aspect according to the eleventh aspect of the description, it is characterized in that a granular particulate recovery unit **(40)** is disposed downstream, relative to the direction of flow of the air to be treated, of the electric discharge device **(20)**.

In the twelfth aspect of the description, the granular particulates **(22),** which have traveled further ahead of the discharge electrode **(21)**, can be recovered by the granular particulate recovery unit **(40).**

In a thirteenth aspect according to the eleventh aspect of the description, it is characterized in that the granular particulate ejector **(24)** is configured such that the granular particulates **(22)** are ejected in a direction opposite to the direction of flow of the air to be treated.

In the thirteenth aspect of the description, the direction of flow of the air to be treated and the direction of ejection of the granular particulates **(22)** from the granular particulate ejector **(24)** are opposite to each other. This results in improvement in the efficiency of contact between the air to be treated and the granular particulates **(22).**

In a fourteenth aspect according to the eleventh aspect of the description, it is characterized in that a dust collecting means **(54)** is disposed upstream, relative to the direction of flow of the air to be treated, of the electric discharge device **(20)**.

In the fourteenth aspect of the description, dust particles present in the air to be treated are collected by the dust collecting means **(54)**. As a result, such dust particles are prevented from adhering to the electric discharge device **(20)**, to the discharge electrode **(21),** and to the granular particulate ejector **(24)**.

In a fifteenth aspect according to the eleventh aspect of the description, it is characterized in that a catalytic treatment part **(55)** is disposed downstream, relative to the direction of flow of the air to be treated, of the electric discharge device **(20)**.

In the fifteenth aspect of the description, the air to be treated, after having been treated in the electric discharge device **(20)**, is passed through the catalytic treatment part **(55)**. In the catalytic treatment part **(55)**, the odorous component remaining in the air to be treated is oxidatively decomposed by the catalyst. In addition, upon the flowing of active species generated by the electric discharge device **(20)** through the catalytic treatment part **(55)**, the catalytic effect of the catalytic treatment part **(55)** is enhanced, thereby promoting the efficiency of decomposition of the odorous component.

In a sixteenth aspect according to the eleventh aspect of the description, it is characterized in that an adsorptive treatment part **(57)** is disposed downstream, relative to the direction of flow of the air to be treated, of the electric discharge device **(20).**

In the sixteenth aspect of the description, the air to be treated, after having been treated in the electric discharge device **(20),** is passed through the adsorption treating part **(57).** In the adsorptive treatment part **(57)**, the odorous component remaining in the air to be treated is removed by adsorption on the adsorbent such as active carbon or the like.

In a seventeenth aspect according to the eleventh aspect of the description, it is characterized in that there is provided a temperature controlling means for controlling the temperature of the air to be treated.

In the seventeenth aspect of the description, with the generation of electric discharge in the electric discharge device **(20)**, the odorous component present in the air to be treated is removed and, in addition, the temperature of the air to be treated is controlled by the temperature controlling means. That is, the air purification device operates as an air conditioning system having a deodorizing function.

### ADVANTAGEOUS EFFECTS OF THE ASPECTS OF THE DESCRIPTION

In accordance with the first aspect of the description, the granular particulates serve as the counter electrode **(22),** whereby the generation of electric sparking is prevented by the action of a space created between the granular particulates, thereby making it possible to stabilize the state of electric discharge. Accordingly, the applying of the electric discharge device **(20)** to the air purification device **(10)** makes it possible to stabilize also the performance of the air purification device **(10).**

In accordance with the second aspect of the description, the discharge electrode **(21)** is formed by a plurality of needle-shaped electrodes **(21a)**. This arrangement makes it becomes possible to further stabilize the state of electric discharge, and the applying of the electric discharge device **(20)** to the air purification device **(10)** makes it possible to stabilize also the performance of the air purification device **(10)**. In addition, the arrangement of using a plurality of needle-like electrodes **(21a)** as the discharge electrode **(21)** provides an expanded electric discharge region, whereby the generation amount of active species is increased to achieve improvement in the performance of treatment.

In accordance with the third aspect of the description, for example, the discharge electrode **(21)** serves as a positive pole and the granular particulate ejector **(24)** serves as a negative pole. This arrangement causes repetition of a series of cycles (i.e., the generation of an electron avalanche from the granular particulate **(22)** ejected form the granular particulate ejector **(24)**, the advance of a leader from the discharge electrode **(21)**, the extinction of the leader, the generation of an electron avalanche from the granular particulate **(22)** and so on), whereby no electric sparking is produced and, as a result, the state of streamer discharge becomes stabilized.

And, pollutants are decomposed by active species (such as fast electrons or the like) in a discharge space plasmized by streamer discharge. Streamer discharge activates O₂ and N₂ constituting air to produce radicals (such as OH). Such radicals also decompose pollutants.

In accordance with the fourth aspect of the description, the liquid droplets **(22)** serve as the counter electrode **(22)**, whereby the generation of electric sparking is prevented by the action of a space created between the liquid droplets **(22)**. This makes it possible to stabilize the state of electric discharge. Accordingly, the applying of the electric discharge device **(20)** to the air purification device **(10)** stabilizes also the performance of the air purification device (10).

In addition, with respect to the generation of radicals by streamer discharge, it is known that OH radicals having strong oxidative power are produced with the aid of the presence of H₂O. Accordingly, if streamer discharge is generated while spraying the liquid (water) droplets **(22)**, this leads to an increase in the amount of generation of OH radicals. This is extremely effective for the decomposition of pollutants.

Furthermore, the electric discharge device **(20)** has a function as a scrubber because it sprays the liquid droplets **(22)**. Accordingly, in addition to the direct decomposition of part of the odorous component by streamer discharge generated by the electric discharge device **(20)**, the remaining odorous component can be collected by the function of the scrubber. Accordingly, it becomes possible to satisfactorily treat the odorous component.

In addition, in the conventional streamer discharge, there is the possibility that, if oxides generated or pollutants adhere to the discharge electrode **(21)**, this may stop electric discharge or give rise to electric sparking. However, if, as in the description, streamer discharge is initiated, with the liquid (water) droplets **(22)** serving as the counter electrode **(22)**, this prevents the arising of such problems. The reason is as follows. Firstly, during the electric discharge, most of the liquid droplets **(22)** are diffused backwardly beyond the discharge electrode **(21)** and some liquid droplets **(22)** adhere to the discharge electrode **(21)**. The liquid droplets **(22)** adhering to the discharge electrode **(21)** are pulled towards the tip of the discharge electrode **(21)** by ionic wind generated with the generation of electric discharge and, as a result, a film of water is formed on the tip of the discharge electrode **(21)** (see Figure **3(A)**). Consequently, electric discharge is generated to the liquid droplets **(22)** from the water film. As described above, the tip of the discharge electrode **(21)** is covered with a film of water and electric discharge is initiated over the water film, as a result of which the discharge electrode **(21)** is cooled and no degradation due to oxidation occurs. In addition, there is a constant supply of new water at the liquid droplet **(22),** and contaminants are scattered away by electric discharge together with water. Consequently, the tip of the discharge electrode **(21)** is free also from the adhesion of contaminants. Consequently, electric discharge is stably generated and the service life of the discharge electrode **(21)** extends. Here, materials having good staying power such as tungsten are generally used to form the discharge electrode **(21)** for streamer discharge. However, in the description, the liquid droplets **(22)** are used as the counter electrode **(22)**, which makes it possible to stably use materials, inferior in durability to stainless or the like but superior in resistance to corrosion, over a long period of time.

In addition, there is a technique known in the art in which electric discharge is generated to water stored in a container so that decomposition products are absorbed in the water (for example, see JP-A-2002-306925). In this case, however, the surface area of water is small and the efficiency of gas-liquid contact is poor. On the other hand, in the description, since streamer discharge is generated to the liquid droplets **(22),** the surface area of water increases thereby enhancing the efficiency of gas-liquid contact to improve the absorption efficiency of decomposition products. Especially, in the case where insoluble gas including oil mist is a target for treatment, this insoluble gas is oxidatively decompressed to change to soluble form and is absorbed in water, thereby making it possible to achieve improvement of the deodorizing effect. In addition, in the description, even when electric discharge is generated in an atmosphere containing combustible oil, there is no possibility of fire ignition because electric discharge is generated to the liquid droplets **(22)**.

In accordance with the fifth aspect of the description, electric discharge (streamer discharge) can be generated in stable manner in the electric discharge device **(20)** which is configured such that the granular particulate ejector **(24)** and the discharge electrode **(21)** are situated face to face with each other.

In accordance with the sixth aspect of the description, the granular particulates **(22)** are ejected towards a hollow circular conical region around the discharge electrode **(21)** from the granular particulate ejector **(24)**. Accordingly, streamer discharge is formed stably with a flared spread.

More specifically, if the grain size of the granular particulates (liquid droplets) is in the range of about 1 to 20 µm, this setting is optimum to render the shape of spray pattern hollow. This spray pattern shape is determined by the balance of the inertia force of the granular particulate and the ionic wind (Figure **3(B)****)** and the shape of spray pattern is made hollow by the generation of ionic wind towards the granular particulate from the discharge electrode. If the granular particulate is smaller than the aforesaid range and light, it will be blown away by ionic wind. On the other hand, if the granular particulate is larger than the aforesaid range and heavy, this impedes the shape of spray pattern to become hollow because the inertial force is great. And, if the shape of spray pattern becomes hollow, this allows electric discharge to have a flared spread.

In accordance with the seventh aspect of the description, the granular particulates **(22)** are ejected towards a solid circular conical region including the discharge electrode **(21)** from the granular particulate ejector **(24)**. Accordingly, streamer discharge is formed into a column shape with almost no flared spread.

In accordance with the eighth aspect of the description, the granular particulates **(22)** are ejected substantially linearly from the granular particulate ejector **(24)** and streamer discharge is generated to the flow of the granular particulates **(22)** from the discharge electrode **(21)** situated lateral to the granular particulate flow.

In accordance with the ninth aspect of the description, the granular particulates **(22)** ejected from the granular particulate ejector **(24)** can be recovered in the granular particulate recovery container **(45)**.

In accordance with the tenth aspect of the description, the granular particulates **(22)** recovered by the granular particulate recovery container **(45)** can be ejected again from the granular particulate ejector **(24)** for reuse thereof.

In accordance with the eleventh aspect of the description, the electric discharge device **(20)**, in which electric discharge is generated between the granular particulate **(22)** ejected from the granular particulate ejector **(24)** and the discharge electrode **(21)**, is employed in an air purification device. This makes it possible to stabilize the state of electric discharge (especially, streamer discharge) and to stably carry out, with the aid of various active species contained in low temperature plasma gas in the electric discharge field, treatment of air to be treated. In other words, the level of performance of the air purification device **(10)** can highly be stabilized.

In accordance with the twelfth aspect of the description, the granular particulate recovery unit **(40)** is disposed downstream, relative to the direction of flow of the air to be treated, of the electric discharge device **(20)**. This arrangement makes it possible for the granular particulate recovery unit **(40)** to recover the granular particulates **(22)** that have flowed further ahead of the discharge electrode **(21)**, whereby the granular particulates **(22)** recovered can be reused.

In accordance with the thirteenth aspect of the description, the direction of flow of the air to be treated and the direction of ejection of the granular particulates **(22)** are made opposite to each other. This arrangement makes it possible to enhance the efficiency of contact between the air to be treated and the granular particulates **(22)**. Accordingly, especially in the case where the granular particulate is in the form of a liquid droplet, it is possible for the liquid droplet **(22)** to absorb therein the odorous component present in the air to be treated, thereby achieving improvement in the performance of removal of the odorous component.

In accordance with the fourteenth aspect of the description, the dust collecting means **(54)** is disposed upstream of the electric discharge device **(20)**. This arrangement makes it possible to prevent dust particles and other substances present in the air to be treated from adhering to the discharge electrode **(21)** and the granular particulate ejector **(24)** in the electric discharge device **(20)**. Accordingly, it is further ensured that the discharge electrode **(21)** and the granular particulate ejector **(24)** are protected from contamination, thereby making it possible to further stabilize the state of electric discharge by the electric discharge device **(20)**.

In accordance with the fifteenth aspect of the description, the catalytic treatment part **(55)** is disposed downstream of the electric discharge device **(20)**. This arrangement enhances the activation of the catalytic treatment part **(55)**, thereby ensuring the further removal of the odorous component or the like present in the air to be treated. Accordingly, the performance of the air purification device can be enhanced effectively.

In accordance with the sixteenth aspect of the description, the adsorptive treatment part **(57)** is disposed downstream of the electric discharge device **(20)**. This arrangement ensures that the odorous component of low-concentration or the like still remaining in the air to be treated can be removed without fail. Accordingly, the performance of the air purification device can be effectively made stable.

In accordance with the seventeenth aspect of the description, the provision of the temperature controlling means for controlling the temperature of the air to be treated makes it possible to not only remove the odorous and noxious components from the air to be treated but also provide, for example, room air conditioning with temperature-controlled air. To sum up, in accordance with the seventeenth aspect of the description, it is possible to provide an air cleaner (air conditioning system) capable of cleansing room air with high efficiency and, in addition, capable of providing room air conditioning and so on.

### BRIEF DESCRIPTION OF THE DRAWINGS

Figure **1** is a block diagram of a kitchen air exhaust system which uses an electric discharge device and an air purification device, both according to a first embodiment of the description.
Figure **2** is a schematic block diagram of an ionization part **(30)**.
Figure **3**, comprised of Figure **3(A)** and Figure **3(B)**, is an explanatory diagram illustrating the mechanism of streamer discharge.
Figure **4** is a block diagram showing an electric discharge device according to a second embodiment of the description.
Figure **5** is a block diagram showing a modification of the second embodiment.
Figure **6** is a block diagram showing another embodiment of the electric discharge device.
Figure **7** is a block diagram showing still another embodiment of the electric discharge device.
Figure **8 (A)** is a development view of the discharge electrode of Figure **7** and Figure **8(B)** is an external view thereof.
Figure **9** is a schematic block diagram of an air purification device according to a third embodiment of the present invention.
Figure **10** is a block diagram illustrating a modification of the third embodiment.

### REFERENCE NUMERALS IN THE DRAWINGS

- 1: kitchen exhaust system
- 10: air purification device
- 20: electric discharge device
- 21: discharge electrode
- 21a: needle-shaped electrode
- 22: liquid droplet (granular particulate, counter electrode)
- 23: electric power supply
- 24: liquid droplet ejector (granular particulate ejector)
- 40: granular particulate recovery unit
- 45: granular particulate recovery container
- 54: dust collecting filter
- 55: catalytic treatment part
- 60: granular particulate circulating mechanism

### BEST MODE FOR CARRYING OUT THE INVENTION

Hereinafter, preferred embodiments of the present invention will now be more fully described with reference to the accompanying drawings.

### FIRST EMBODIMENT OF THE INVENTION

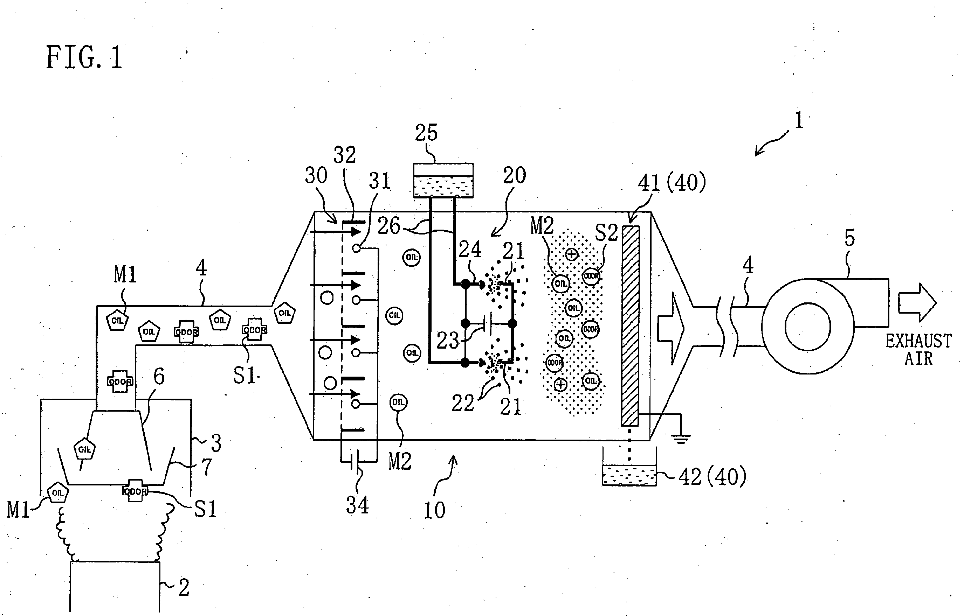

A first embodiment of the present invention is an example of the application of an air purification device **(10)** including an electric discharge device **(20)** of the present invention to an exhaust duct by which exhaust air in a restaurant kitchen is expelled outdoors. Figure **1** is a schematic of a kitchen exhaust system **(1)** including the air purification device **(10)**.

Referring to Figure **1**, there is shown an exhaust hood **(3)** mounted above a cooking appliance **(2)** arranged in the kitchen. One end (indoor side end) of an exhaust duct **(4)** extending to outside the building is connected to the exhaust hood **(3)**. The other end (outdoor side end) of the exhaust duct **(4)** is connected to an exhaust fan **(5)**. By this configuration, kitchen exhaust air, i.e., air to be treated, is released outdoors after being passed sequentially through the exhaust hood **(3),** then through the exhaust duct **(4)**, and then through the exhaust fan **(5).**

A grease filter **(6)** for collecting an oil mist **(M1)** and dust (not shown) contained in the kitchen exhaust air is disposed in the exhaust hood **(3)**. In addition, an oil pan **(7)** for receiving waste oil is placed below the grease filter **(6)**.

The kitchen exhaust air contains, in addition to the oil mist (M1) and dust, an odorous component (S1). And, the air purification device **(10)** of the present invention is mounted in the middle of the exhaust duct **(4)** so that the oil mist (M1) and the odorous component (S1) past the grease filter **(6)** are removed.

The air purification device **(10)** has, as a main constituent element thereof, a streamer discharge part (electric discharge device) **(20)** for decomposing the odorous component (S1). The streamer discharge part **(20)** decomposes the odorous component or the like with the aid of active species produced by streamer discharge. In addition, the streamer discharge part **(20)** serves also as a scrubber for dispersing a cleaning liquid (water) to the kitchen exhaust air so that oil mist and dust particles are collected by droplets of the liquid. An ionization part **(30)** for electrically charging the dust (dust particles) and the oil mist (M1) present in the kitchen exhaust air is disposed upstream of the streamer discharge part **(20)**. In addition, a demister **(41)** for collecting the electrically-charged dust particles is disposed downstream, relative to the direction of flow of the kitchen exhaust air, of the streamer discharge part **(20)**. A recovery tank **(42)** is disposed so as to collect liquid droplets adhering to the demister **(41)** after ejection from the streamer discharge part **(20)** when they fall from the demister **(41).** The demister **(41)** and the recover tank **(42)** together constitute a liquid droplet collecting device (granular particulate recovery device) **(40)**.

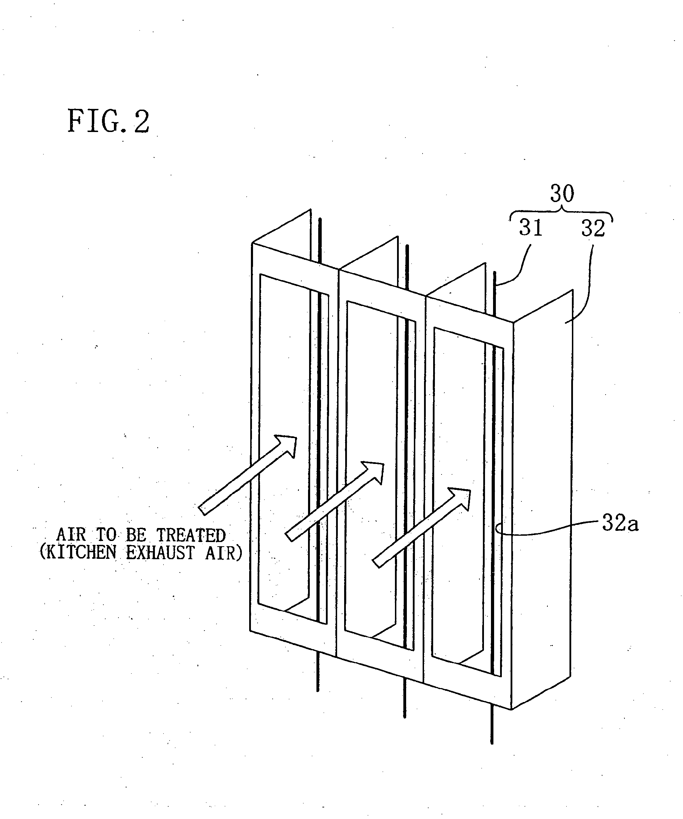

Referring now to Figure **2**, there is perspectively shown a schematic configuration of the ionization part **(30)**. The ionization part **(30)** causes the oil mist (M1) and dust of relatively small size past the grease filter **(6)** to be electrically charged, whereby the collecting of these oil mist/dust particles by the demister **(41)** is facilitated. In the first embodiment, the demister **(41)** is connected to an earth electrode so that the oil mist/dust particles electrically charged are collected because of the difference between their electric potential and the electric potential of the demister **(41)**.

The ionization part **(30)** is composed of a plurality of ionization lines **(31)** and a plurality of counter electrodes **(32).** Being equally spaced, the plurality of ionization lines **(31)** extend from top to bottom of the ionization part **(30)**. Each ionization line **(31)** lies on a single virtual surface in parallel with the other. Each counter electrode **(32)** has, in a front plate-like portion thereof, an opening **(32a)** for ventilation, and is provided, between each ionization line **(31)**, with a partition plate (electrode plate). Each ionization line **(31)** of the ionization part **(30)** is connected to the positive pole of an electric power supply **(34)** for the ionization part and each counter electrode **(32)** is connected to the negative pole of the electric power supply **(34)**. In addition, the ionization line **(31)** and the counter electrode **(32)** are electrically insulated from each other by an insulator (not shown).

The streamer discharge part **(20)** includes a discharge electrode **(21)**, a counter electrode **(22)**, and an electric power supply **(23)** for the electric discharge device for providing a difference in electric potential between both the electrodes **(21, 22)**. The streamer discharge part **(20)** generates streamer discharges from the discharge electrode **(21)** towards the counter electrode **(22)**. The streamer discharge part **(20)** is provided, in addition to the discharge electrode **(21)** formed by a needle-shaped electrode, with a liquid droplet ejector (granular particulate ejector) **(24)** for spraying droplets of liquid. The liquid droplets ejected from the liquid droplet ejector **(24)** towards the discharge electrode **(21)** are used as the counter electrode (**22)**. The liquid droplet ejector **(24)** is configured such that the liquid droplets **(22)** are ejected towards a circular conical region around the discharge electrode **(21).**

The mutual relationship in position between the discharge electrode **(21)** and the liquid droplet ejector **(24)** is determined so that, according to the difference in electric potential between the discharge electrode **(21)** and the counter electrode **(22)** (composed of liquid droplets), there is generated a streamer discharge between the discharge electrode **(21)** and the liquid droplets **(22)**. In addition, the direction of ejection of the liquid droplets **(22)** from the liquid droplet ejector **(24)** is substantially oriented to the discharge electrode **(21)** and the tip of the discharge electrode **(21)** is substantially oriented to the liquid droplet ejector **(24).** In other words, the discharge electrode **(21)** and the liquid droplet ejector **(24)** are arranged face to face with each other. In addition, the shape of ejection pattern of the liquid droplets **(22)** from the liquid droplet ejector **(24)** is determined to have a hollow circular conical shape centered on the discharge electrode **(21).**

More specifically, if the grain diameter of the liquid droplets is set to fall within the range of about 1 to about 20 µm, this setting is suitable for providing a shape of spray pattern which is a hollow circular cone. The spray pattern shape is determined by the balance between the liquid droplet's inertial force and the ionic wind, and the generation of a flow of ionic wind from the discharge electrode towards the liquid droplets provides a hollow spray pattern shape (Figure **3(B)****).** The liquid droplets, if their grain diameter falls below the aforesaid range and they are light, will be blown off by ionic wind. Conversely, if the liquid droplets have a grain diameter exceeding the aforesaid range and are heavy, this impedes the formation of a hollow spray pattern shape because the inertial force is great. When the spray pattern shape becomes hollow, this provides an electric discharge with a flared spread.

The streamer discharge part **(20)** is provided with a water tank **(25)** for holding cleaning liquid (water) and a water supply pipe **(26)** for the supply of liquid (water) from the water tank **(25)** to the liquid droplet ejector **(24)**. In the streamer discharge part **(20)**, the positive pole of the electric power supply **(23)** for the electric discharge device is connected to the discharge electrode **(21)** and the negative pole thereof is connected to the liquid droplet ejector's **(24)** side.

### RUNNING OPERATION

Next, the action of purifying kitchen exhaust air during the operation of the kitchen exhaust system **(1)** will be described below.

Kitchen exhaust air is drawn into the exhaust hood **(3)** by the exhaust fan **(5)** connected to the exhaust duct **(4)**. Since the grease filter **(6)** is mounted in the exhaust hood **(3),** most of the oil mist **(M1)** and the dust contained in the kitchen exhaust air are collected by the grease filter **(6).** More specifically, the oil mist **(M1)** having a relatively large grain diameter (about 10 µm or above) and occupying about 80% to about 90% of the total amount of the oil mist **(M1)** in the kitchen exhaust air is collected by the grease filter **(6).** Therefore, the kitchen exhaust air past the grease filter **(6)** is in such a state that the fine oil mist (M1) having a grain diameter of less than about several micrometers still remains therein, but the amount thereof is small. In addition, since the odorous component (S1) contained in the kitchen exhaust air is the form of a gas molecule, it flows through the exhaust duct **(4)** without being removed by the grease filter **(6).**

During the passage through the air purification device **(10),** the kitchen exhaust air is subjected to treatment such as the removal of the oil mist (M1) and dust particles and the decomposition of the odorous component (S1).

More specifically, the oil mist (M1) and the dust are electrically charged in the ionization part **(30)**. The manner of this change is represented, in the figure, by the change in shape of the oil mist, that is, from the pentagonal oil mist (M1) to a circular oil mist (M2).

In the streamer discharge part **(20)**, cleaning liquid (water) is sprayed from the liquid droplet ejector **(24)**. Since the oil mist (M2) and the dust change from hydrophobic to hydrophilic form by the action of streamer discharge, they are absorbed in droplets (D) of the cleaning liquid, adhere to the demister **(41)** together with the cleaning liquid, and fall downwards therefrom, and are recovered in the recovery tank **(42)**. In addition, although not shown in the figure, there occurs a separation of the cleaning liquid and the oil in the recovery tank **(42)**, and the oil floats on the surface of the cleaning liquid.

In the streamer discharge part **(20)**, streamer discharge is generated from the tip of each discharge electrode **(21)** towards the liquid droplets **(22)** serving as the counter electrode **(22).** The streamer discharge is formed, by a continuous series of minute electric arcs from the tip of the discharge electrode **(21)** to the liquid droplets **(22)**, as a plasma column with luminescence. Such a minute electric arc spreads in the form of a flare and advances between the tip of the discharge electrode **(21)** and the liquid droplets **(22)**.

More specifically, if the discharge electrode **(21)** serves as a positive pole and the liquid droplet ejector **(24)** serves as a negative pole (see Figure **3(A)**), the liquid droplets **(22)** are induced by an electric field between the discharge electrode **(21)** and the liquid droplet ejector **(24)** and jump out from the liquid droplet ejector **(24)** with negative electric charges. Then, there is produced an electron avalanche from the liquid droplets **(22)** of negative electric charge towards the discharge electrode **(21)** of positive electric charge, as a result of which the liquid droplets **(22)** lose the negative electric charges, thereby entering the electrically neutral state. On the other hand, a minute electric arc (column), which is called a "leader" of positive electric charge, advances towards the liquid droplets **(22)** from the discharge electrode **(21)**. Here, if the positively-charged liquid droplets **(22)** stand still or are travelling towards the liquid droplet ejector **(24)**, this causes the possibility that the leader may further advance with the movement of the liquid droplets **(22)** to become an electric spark. However, the liquid droplets **(22)** travel in a direction moving away from the liquid droplet ejector **(24)** and, besides, there exists a space between the liquid droplets **(22)** which space functions as an insulator. As a result, the leader will not advance further. That is, the leader is unable to change to an electric spark. The streamer discharge is an electric discharge that repeats a series of cycles (i.e., "electron avalanche"→"leader formation"→ "leader extinction"→"electron avalanche and so on). In the first embodiment, such a series of cycles is repeated in ideal form, whereby streamer discharge is stably formed as a flared plasma column with luminescence

By the aforesaid streamer discharge, low temperature plasma gas is generated in the electric discharge filed. The gas thus generated contains, as active species, fast electrons, ions, ozone, radicals such as hydroxyl radicals, and other excited molecules (e.g., excited oxygen molecules, excited nitrogen molecules, excited water molecules et cetera). And, these active species starts to flow in a direction downstream of the flow of kitchen exhaust air.

When the kitchen exhaust air is flowing through the streamer discharge part (20), a part of the odorous component (S1) contained in the kitchen exhaust air is decomposed directly by the active species, changes to CO₂ and H₂O, and becomes odorless.

On the other hand, the rest of the odorous component (S1) bonds to active species such as electrons and various excited molecules and changes from hydrophobic (lipophilic) to hydrophilic form. The manner of this change is represented, in the figure, by the change in shape of the odorous component, that is, from the odorous component (S1) in the shape of a cross to an odorous component (S2) in the shape of a circle. The odorous component (S2), which has changed to hydrophilic form, dissolves in the liquid droplets **(22)** of the cleaning liquid (water) in the streamer discharge part **(20)** serving also as a scrubber. In addition, in the streamer discharge part (scrubber) **(20)**, the remaining active species are also collected in the liquid droplets **(22)** of the cleaning liquid. Accordingly, the hydrophilic odorous component (S2) remaining undecomposed and the active species which have not used for the decomposition of the odorous component are also collected in the recovery tank **(42)**.

Consequently, from the streamer discharge part (scrubber) **(20)** to the recovery tank **(42)**, the odorous component (S1) is decomposed by the action of the active species during the time from when the liquid droplets **(22)** of the cleaning liquid are in the middle of dropping from the demister **(41)** until when the liquid droplets **(22)** are recovered in the recovery tank **(42)**.

And, the kitchen exhaust air, from which the odorous component (S1) has been removed together with the oil mist (M1) and the dust, is released outdoors through the outlet of the air purification device **(10)** by the action of the exhaust fan **(5)**. At this time, since the odorous components (S1, S2) have already been removed from the exhaust air, there is no emission of odor to outside the building.

### ADVANTAGEOUS EFFECT OF THE FIRST EMBODIMENT

In accordance with the first embodiment, the counter electrode **(22)** is in the form of liquid droplets. As a result of this arrangement, the generation of electric sparking can be prevented by a space created between the liquid droplets **(22)**. And, thanks to the repetition of a series of cycles (i.e., the occurrence of an electron avalanche from the liquid droplet **(22)** ejected from the liquid droplet ejector **(24)**, the advance of a leader from the discharge electrode **(21),** the extinction of the leader, the occurrence of an electron avalanche from the liquid droplet **(22)** and so on) owing to the arrangement that the discharge electrode **(21)** serves as a positive pole and the liquid droplet ejector **(24)** serves as a negative pole, and also thanks especially to the arrangement that the liquid droplet ejector **(24)** and the discharge electrode **(21)** are oriented face to face with each other, the occurrence of electric sparking is prevented whereby the state of streamer discharge is stabilized. Accordingly, the applying of the electric discharge device **(20)** to the air purification system **(10)** makes it possible that the level of performance of the air purification device **(10)** can highly be stabilized.

In addition, the liquid droplets **(22)** are ejected from the liquid droplet ejector **(24)** towards a hollow circular conical region around the discharge electrode **(21)**. As a result of such arrangement, streamer discharge is stably formed having a flared spread.

And, pollutants are decomposed by active species, such as fast electrons et cetera, in a discharge space plasmized by streamer discharge. In addition, streamer discharge activates O₂ and N₂ constituting air to produce radicals such as OH. Such radicals also decompose pollutants. With respect to the generation of radicals, it is known that OH radicals having strong oxidative power are produced with the aid of the presence of H₂O. Accordingly, if streamer discharge is generated while spraying the liquid (water) droplets **(22)**, this leads to an increase in the amount of generation of OH radicals. This is extremely effective for the decomposition of pollutants.

Furthermore, the electric discharge device **(20)** initiates streamer discharge, whereby a part of the odorous component (S1) is decomposed by the streamer discharge and, in addition, the rest of the odorous component (S1) changes from hydrophobic to hydrophilic form, thereby making it possible for the electric discharge device **(20)** to capture the odorous component (S2) together with the remaining active species by using a function as a scrubber. Accordingly, since the action of decomposition of the odorous component (S2) can be obtained also by the scrubber, this makes it possible to satisfactorily treat the odorous component (S1) contained in the kitchen exhaust air.

In addition, in the conventional streamer discharge, there is the possibility that, if oxides generated or pollutants adhere to the discharge electrode **(21),** this may stop electric discharge or give rise to electric sparking. However, if, as in the first embodiment, streamer discharge is initiated, with the liquid (water) droplets **(22)** serving as the counter electrode **(22)**, this prevents the arising of such problems. More specifically, during the electric discharge, most of the liquid droplets **(22)** are diffused backwardly beyond the discharge electrode **(21)** while some liquid droplets **(22)** adhere to the discharge electrode **(21)**. These liquid droplets **(22)** adhering to the discharge electrode **(21)** are attracted towards the tip of the discharge electrode **(21)** by ionic wind generated with the generation of electric discharge and, as a result, a film of water is formed on the tip of the discharge electrode **(21)** (see Figure **3(A)**). Consequently, electric discharge is generated to the liquid droplets **(22)** from the film of water. As described above, the tip of the discharge electrode **(21)** is covered with a film of water and electric discharge occurs over the film of water, as a result of which the discharge electrode **(21)** is cooled and no degradation due to oxidation occurs. In addition, there is a constant supply of new water as the liquid droplets **(22)**, and contaminants are scattered away by electric discharge together with the water. Consequently, the tip of the discharge electrode **(21)** is free also from the adhesion of contaminants. This provides stable electric discharge and therefore increases the service life of the discharge electrode **(21)**. Here, materials having good staying power such as tungsten are generally used to form the discharge electrode **(21)** for streamer discharge. However, water is used in the first embodiment, which makes it possible to stably use materials, inferior in durability to stainless or the like but superior in resistance to corrosion, over a long period of time.

In addition, there is a technique known in the art in which technique electric discharge is generated to water held in a container so that decomposition products are absorbed in the water (for example, see JP-A-2002-306925). In this case, however, the surface area of water is small and the efficiency of gas-liquid contact is poor. On the other hand, in the first embodiment, streamer discharge is generated to the liquid droplets **(22)**. This achieves an increase in the surface area of water, whereby the efficiency of gas-liquid contact is enhanced to improve the efficiency of absorption of the decomposition product. Especially, insoluble gas containing oil mist or the like is oxidatively decomposed and converted to soluble form. And, the now soluble gas is absorbed in the water. As a result, the effect of deodorizing is improved. In addition, in the present embodiment, electric discharge is initiated in an atmosphere containing combustible oil, but there is no possibility for the occurrence of fire ignition because electric discharge is generated to the liquid droplets **(22).**

### MODIFICATION OF THE FIRST EMBODIMENT

In the first embodiment, it is arranged such that the shape of spray pattern of the liquid droplets **(22)** from the liquid droplet ejector **(24)** has a hollow circular conical shape. Alternatively, the shape of spray pattern may have a solid circular conical shape. As a result of such arrangement, in the modification, streamer discharge is formed in a column-shaped region with a less flared spread in comparison with the first embodiment in which streamer discharge is formed in a region with a flared spread.

However, the shape of spray pattern is limited to neither a hollow circular conical shape nor a solid circular conical shape. The shape of spray pattern may have different shapes other than those described above. The granular particulate ejector **(24)** may be implemented by either a gas-liquid two-fluid nozzle, a ultrasonic atomizer, or an electrostatic atomizer.

### SECOND EMBODIMENT OF THE INVENTION

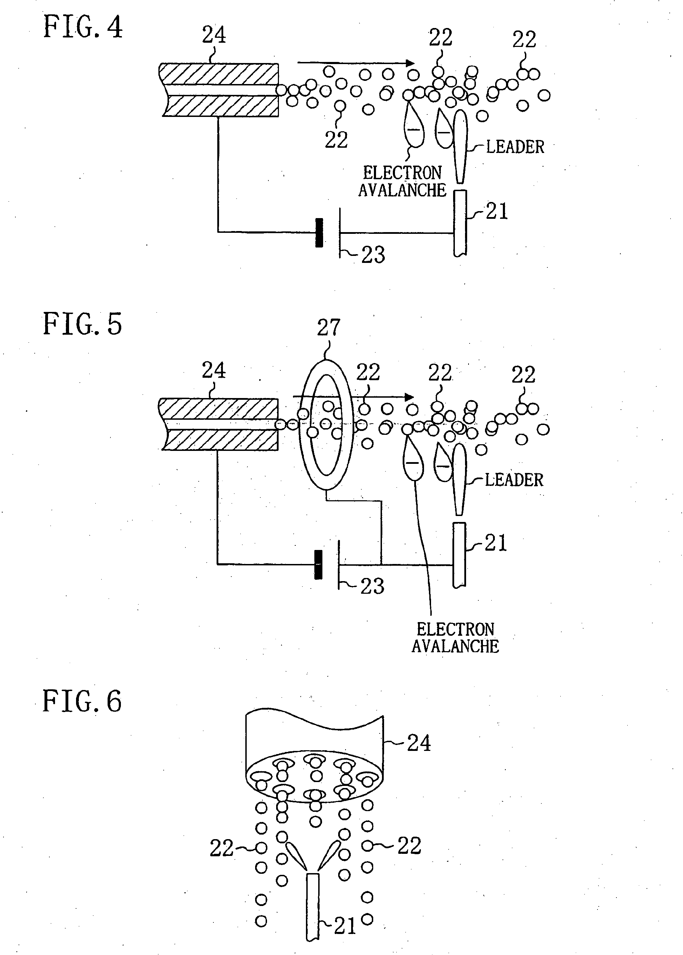

The present invention provides a second embodiment as an example that differs, in the relationship in position between the liquid droplet ejector **(24)** and the discharge electrode **(21)**, from the first embodiment, as shown in Figure 4. More specifically, it is configured such that the liquid droplet ejector **(24)** linearly sprays the liquid droplets **(22)** and, in addition, the tip of the discharge electrode **(21)** is oriented from sideways to the direction of flow of the liquid droplets **(22)** sprayed from the liquid droplet ejector **(24)** (approximately at right angles to the direction of flow of the liquid droplets **(22)**).

Also according to the above arrangement, in order to generate a streamer discharge to the liquid droplets **(22)**, a series of cycles (i.e., the generation of an electron avalanche from the liquid droplet **(22)** ejected from the liquid droplet ejector **(24),** the advance of a leader from the discharge electrode **(21)**, the extinction of the leader, the generation of an electron avalanche from the liquid droplet **(22)** and so on), is repeated. Accordingly, the state of electric discharge becomes stabilized, thereby achieving improvements in the performance of air purification if the electric discharge device **(20)** is applied to the air purification device **(10).**

### MODIFICATION OF THE SECOND EMBODIMENT

It is advisable that the electric discharge device **(20)** of the second embodiment is provided with a ring-shaped guide member **(27)** through which hollow space the liquid droplets **(22)** sprayed from the liquid droplet ejector **(24)** are passed, as shown in Figure 5. As a result of such arrangement, an electron avalanche from the liquid droplet **(22)** trends to take place, whereby the state of streamer discharge becomes more stabilized in comparison with the example of **Figure 4****.**

### THIRD EMBODIMENT OF THE INVENTION

The present invention provides a third embodiment as an example of the application of an air purification device, which is provided with the electric discharge device **(20)** of the present invention, to an air cleaner **(10)** for general household use. The air cleaner **(10)** is for cleansing an indoor space by removing substances present in the room air such as odorous and noxious components, dust particles and so on.

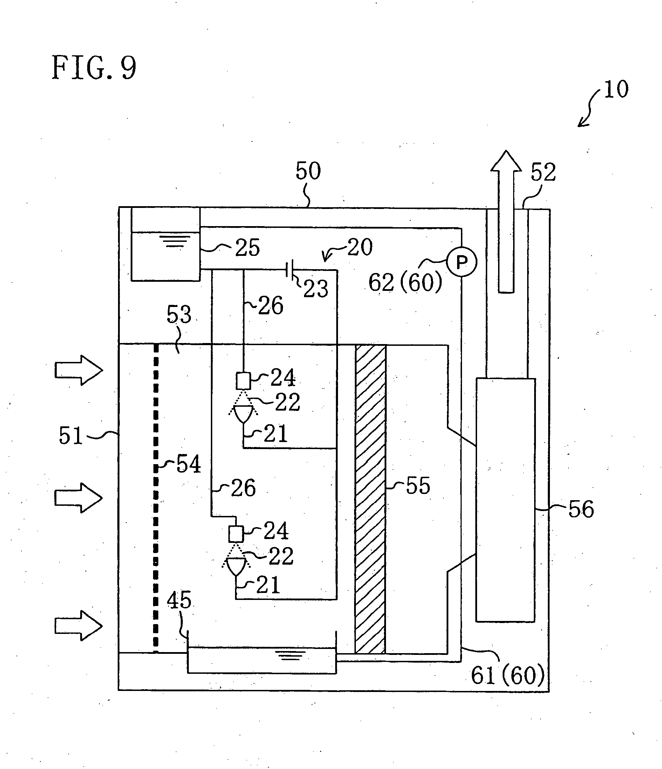

As shown in Figure 9, the air cleaner **(10)** has a rectangular casing **(50).** The casing **(50)** is provided, in its front surface, with an air intake opening **(51)**, and an air delivery opening **(52)** is formed in the upper surface. And, there is provided in the casing **(50)** an air passageway **(53)** extending from the air intake opening **(51)** to the air delivery opening **(52)**. Room air, i.e., air to be treated, is passed through the air passageway **(53).**

A dust collecting filter **(54)**, a streamer discharge part **(20)**, a catalytic treatment part **(55)**, and an air supply fan **(56)** are disposed, sequentially in the order from upstream to downstream side, in the air passageway **(53)**.

The dust collecting filter **(54)** constitutes a dust collecting means for physically collecting dust particles of relatively large size present in the room air. The streamer discharge part **(20)** generates a streamer discharge, whereby odorous and noxious components and the like are oxidatively decomposed by active species generated by the streamer discharge, as in the case of each of the aforesaid embodiments. The catalytic treatment part **(55)** is constructed such that it supports, on its substrate surface, a catalyst. For example, catalysts of the manganese family and catalysts of the noble metal family may be used as the catalyst that the catalytic treatment part **(55)** can support thereon.

The streamer discharge part **(20)** is provided with a plurality of discharge electrodes **(21)** and a plurality of liquid droplet ejectors **(24)**. Each discharge electrode **(21)** is formed by a needle-shaped electrode and is arranged in the air passageway **(53)** in such an orientation that it extends vertically. Each liquid droplet ejector **(24)** is so arranged above its associate discharge electrode **(21)** as to face the tip thereof.

Each liquid droplet ejector **(24)** is connected through the water supply pipe **(26)** to the water tank **(25)**, as in the aforesaid embodiments. That is, it is configured such that the supply of water held in the water tank **(25)** is provided to each liquid droplet ejector **(24)**. Each liquid droplet ejector **(24)** is configured such that the liquid droplets **(22)** which become granular particulates are ejected downwardly. In addition, the shape of spray pattern of the liquid droplets **(22)** from the liquid droplet ejector **(24)** is determined to have a hollow circular conical shape centered on the discharge electrode **(21)**, as in the first embodiment.

In addition, like the foregoing first embodiment, the streamer discharge part (20) is provided with an electric power supply **(23)** for the electric discharge device. The positive side of the electric power supply **(23)** for the electric discharge device is connected to the discharge electrode **(21).** On the other hand, the negative side of the electric power supply **(23)** for the electric discharge device is connected through the water supply pipe **(26)** to the liquid droplet ejector **(24)**. That is, like the aforesaid first embodiment, the liquid droplets **(22)** sprayed from the liquid droplet ejector **(24)** are electrically negatively charged, and constitute a counter electrode for the discharge electrode **(21)**. In the streamer discharge part **(20)**, there is provided between the discharge electrode **(21)** and the liquid droplet ejector **(24)** an electric potential difference, as in the aforesaid first embodiment. As a result, a streamer discharge in the form of a flare advances towards the liquid droplets **(22)** from the tip of the discharge electrode **(21)**.

The streamer discharge part **(20)** of the third embodiment is provided with a water recovery container **(45)** and a water circulating mechanism **(60)**.

The water recovery container (granular particulate recovery container) **(45)** is situated underneath the liquid droplet ejector **(24)** and is mounted on the bottom of the casing **(50)**. The water recovery container **(45)** is so configured as to be capable of recovery of the liquid droplets **(22)** ejected from the liquid droplet ejector **(24).** In addition, the water recovery container **(45)** is so configured as to be capable of attachment to and detachment from the casing **(50).** On the other hand, the casing **(50)** has an access opening through which to pull out the water recovery container **(45)** to outside the casing **(50)**, and an openable and closable cover for the access opening (diagrammatical representation is omitted). That is, the water recovery container **(45)** is so configured as to be capable of freely being taken out from and inserted into the casing **(50)**.

The water circulating mechanism (granular particulate circulating mechanism) **(60)** is configured such that it returns the liquid (water) droplets **(22)**, recovered in the water recovery container **(45)**, to the liquid droplet ejector's **(24)** side. The water circulating mechanism **(60)** is provided with circulation piping **(61)** and a circulation pump **(62)**. One end of the circulation piping **(61)** is connected to the water recovery container **(45)** and the other end thereof is connected to the water tank **(25)**. The circulation pump **(62)** is disposed in the circulation piping **(61).** The circulation pump **(62)** is configured such that it delivers, by pressure, water accumulated in the water recovery container **(45)** to the water tank **(25)**.

### RUNNING OPERATION

The following is a description of the running operation of the air cleaner **(10)**. During the running operation of the air cleaner **(10)**, the air supply fan **(56)** is placed in operation, and room air is introduced through the air intake-opening **(51)** into the air passageway **(53)**.

In the first place, air to be treated is passed through the dust collecting filter **(54)**. The dust collecting filter **(54)** captures dust particles contained in the room air. Thereafter, the room air passes through the vicinity of the discharge electrode **(21)** and the liquid droplet ejector **(24)** in the streamer discharge part **(20)**.

During the operation of the air cleaner **(10)**, the liquid droplets **(22)** are ejected downwardly from each of the liquid droplet ejectors **(24)**. In addition, the electric power supply **(23)** for the electric discharge device provides an electric potential difference between the liquid droplet ejector **(24)** and the discharge electrode **(21)**. As a result, a minute electric arc advances between the discharge electrode **(21)** and the liquid droplets **(22)** such that it spreads upwardly. That is, the streamer discharge part **(20)** of the third embodiment, too, generates an electric discharge that repeats a series of cycles (i.e., "electron avalanche"→"leader formation "leader extinction"→"electron avalanche" and so on), whereby streamer discharge is stably formed as a flared plasma column with luminescence (see **Figure 3(A)**), as in the case of the first embodiment. As a result, in the streamer discharge part **(20)**, fast electrons, ions, ozone, radicals (such as hydroxyl radicals), and other excited molecules (excited oxygen molecules, excited nitrogen molecules, excited water molecules et cetera) are generated in the air passageway **(53)**.

The odorous and noxious components contained in the air to be treated are oxidatively decomposed by the aforesaid active species. In addition, the hydrophilic odorous component or the like contained in the air to be treated is absorbed in the liquid droplets **(22)** ejected from the liquid droplet ejector **(24)**. Furthermore, minute particulates (such as dust particles) contained in the air to be treated are physically absorbed in the liquid droplets (22).

Thereafter, the air to be treated passes, together with active species, through the catalytic treatment part **(55)**. In the catalytic treatment part **(55)**, the odorous component or the like lingering in the air to be treated is oxidatively decomposed by catalyst. In addition, the passage of the active species through the catalytic treatment part **(55)** enhances the catalytic action of the catalytic treatment part **(55)**, whereby the odorous component or the like is oxidatively decomposed to a further extent. The air to be treated, which has been purified in the way as described above, is supplied indoors through the air delivery opening **(52)**.

### ADVANTAGEOUS EFFECTS OF THE THIRD EMBODIMENT

In accordance with the third embodiment, electric discharge is generated towards the liquid droplets **(22)** ejected from the liquid droplet ejector **(24)**, as in the case of the first embodiment. As a result of such arrangement, no electric sparking occurs, whereby the state of streamer discharge becomes stabilized. Accordingly, it is possible to improve the efficiency of air purification of the air cleaner **(10)**.

In the third embodiment, it is arranged such that the dust collecting filter **(54)** is disposed upstream of the streamer discharge part **(20)**. This arrangement prevents the adhesion of dust particles of relatively large size contained in the air to be treated to the discharge electrode **(21)** and to the liquid droplet ejector **(24).** As a result, even if the air cleaner **(10)** is operated over a long period of time, neither the shape of spray pattern of the liquid droplets **(22)** ejected from the liquid droplet ejector **(24)** nor the properties of electric discharge advancing from the discharge electrode **(21)** will not be damaged. Therefore, the state of streamer discharge from the streamer discharge part (40) becomes further stabilized.

In the third embodiment, the catalytic treatment part **(55)** is disposed downstream of the streamer discharge part **(20)**. As a result of such arrangement, it is ensured that the residual odorous component or the like in the air to be treated is removed. Furthermore, the power of decomposition of the odorous component in the catalytic treatment part **(55)** is enhanced by active species generated in the streamer discharge part **(20).** This further ensures the removal of the odorous component remaining in the air to be treated.

In accordance with the third embodiment, the water recovery container **(45)** underlies the liquid droplet ejector **(24)**, thereby ensuring the recovery of water sprayed from the liquid droplet ejector **(24)**. In addition, the water recovery container **(45)** can be put in and taken out from the casing **(50)**, thereby facilitating the maintenance of the water recovery container **(45)**.

In the third embodiment, water recovered in the water recovery container **(45)** is returned to the water tank **(25)** by the water circulating mechanism **(60)** and the water thus recovered is ejected again from the liquid droplet ejector **(24)**. As a result of such arrangement, it is possible to cut down the amount of consumption of the water required for the generation of streamer discharge, thereby reducing the running cost of the air cleaner **(10).**

### MODIFICATION OF THE THIRD EMBODIMENT

As a substitute for the duct collecting filter **(54)** of the third embodiment, other types of dust collecting means (for example, electric dust collectors, cyclone dust collectors, scrubber dust collectors et cetera) may be used. It is also possible for these dust collecting means to capture, on the side upstream of the streamer discharge part **(20)**, dust particles present in the air to be treated, thereby positively ensuring prevention of the adhesion of dust particles or the like to the liquid droplet ejector **(24)** and the discharge electrode **(21)** in the streamer discharge part **(20)**.

As a substitute for the catalytic treatment part **(55)** of the third embodiment, an adsorptive treatment part **(57)** may be disposed downstream of the streamer discharge part **(20).** The adsorptive treatment part **(57)** is configured such that it supports, on the surface of an air distributable substrate thereof, an adsorbent such as active carbon or the like. This configuration makes it possible that the component to be treated, which is still remaining in the air to be treated, is absorption removed by the adsorptive treatment part **(57)** after the passage through the streamer discharge part (20).

In addition, it may be configured such that the spray operation of the liquid droplet ejector **(24)** is forced to a stop upon the detection that the water recovery container **(45)** of the third embodiment is extracted out from within the casing **(50).** This configuration prevents the occurrence of a situation where the liquid droplets **(22)** are accidentally ejected from the liquid droplet ejector **(24)** even after the detachment of the water recovery container **(45)** from the casing **(50).**

In addition, in the third embodiment, there may be provided a water treatment device for the treatment of a noxious component or the like present in the water recovered in the water recovery container **(45)**. As a result of such arrangement, it is prevented that the water quality of the liquid droplets **(22)** ejected from the liquid droplet ejector **(24)** will become degraded, thereby making it possible to achieve the reuse of spray water over a long period of time.

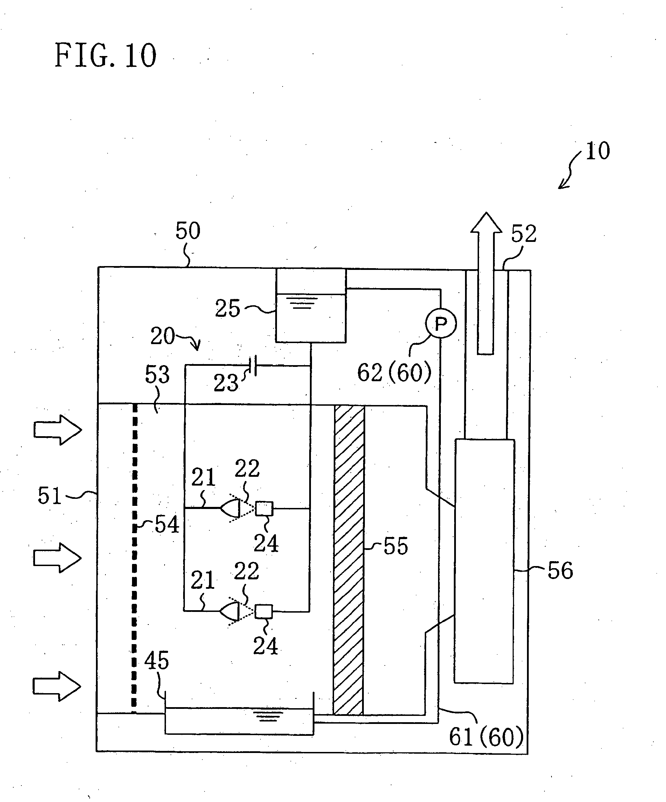

Furthermore, for example, as shown in Figure 10, the liquid droplet ejector **(24)** may be configured such that the liquid droplets (granular particulates) **(22)** are ejected in a direction opposite to the direction of flow of the air to be treated. In this case, the efficiency of contact between the liquid droplets **(22)** ejected from the liquid droplet ejector **(24)** and the air to be treated is improved, thereby making it possible to achieve effective absorption of the air to be treated into the liquid droplets **(22).** Accordingly, it is possible to improve the performance of the air cleaner **(10)**.

Besides, there may be provided, in the air passageway **(53)** of the air cleaner **(10)** of the third embodiment, a temperature controlling means (such as a heat exchanger, a heater, a cooler et cetera) for controlling the temperature of the air to be treated. As a result of such arrangement, it becomes possible to deodorize the air to be treated by streamer discharge generated by the electric discharge device **(20)** and, in addition, to provide air conditioning of an indoor space or the like by heating or cooling the air to be treated. That is, the provision of the electric discharge device **(20)** and the temperature control means to the air cleaner **(10)** makes it possible to constitute an air conditioning system having a deodorizing function of high level.

In addition, each of the electric discharge devices (streamer discharge parts) of the first and second embodiments and their modifications may be employed in the air cleaner **(10)** of the third embodiment.

### OTHER EMBODIMENTS OF THE INVENTION

The foregoing embodiments may be configured as follows.

For example, in the first embodiment, the description has been made in terms of an example where the air purification device **(10)** according to the present invention is applied to the kitchen exhaust system **(1)**. However, the air purification device **(10)** of the present invention may be applied to other than the kitchen exhaust system **(1)**. In addition, the electric discharge device **(20)** of the present invention uses granular particulates such as liquid (water) droplets as the counter electrode **(22)** for the stabilization of electric discharge (streamer discharge), and may be applied to any device other than the air purification device **(10)** as long as it utilizes electric discharge (streamer discharge).

In addition, it may be arranged such that the liquid droplet ejector **(24)** is of the funnel type which is oriented facedown so that the liquid droplets **(22)** drop (as shown in Figure **6**) while the discharge electrode **(21)** is oriented faceup so as to face the funnel type liquid droplet ejector **(24)**. This configuration also provides the same effects that each of the foregoing embodiments does.

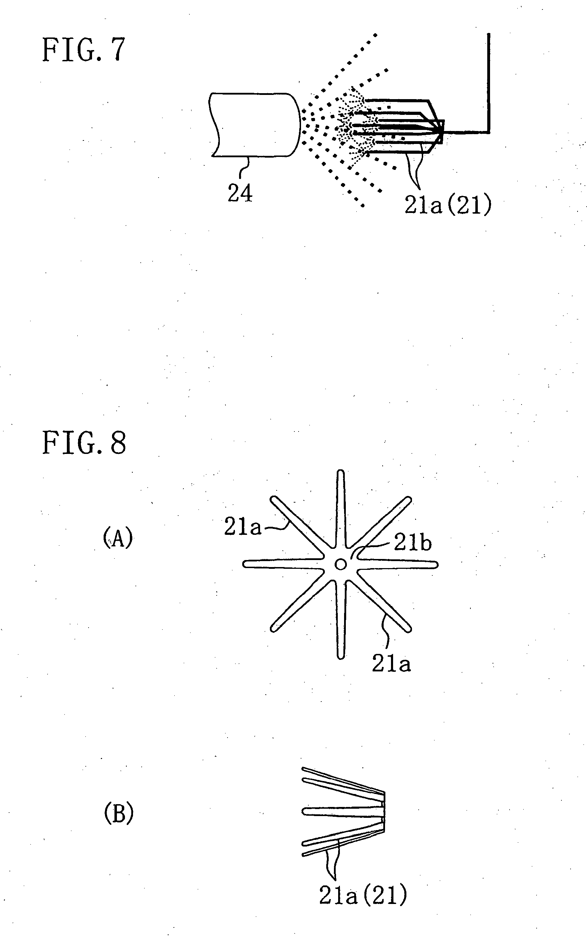

In addition, in each of the first to third embodiments, the description has been made in terms of an example in which a single needle-shaped electrode is used as the discharge electrode **(21).** Alternatively, the discharge electrode **(21)**, as shown in Figures **7** and **8**, may be composed of a plurality of needle-shaped electrodes **(21a).** If, as described above, the discharge electrode **(21)** is composed of a plurality of needle-shaped electrodes **(21a)**, this makes it possible, even when there is a needle-shaped electrode **(21a)** whose state of electric discharge is unstable, to further stabilize the state of electric discharge because of the existence of the other needle-shaped electrodes **(21a)**. Accordingly, the applying of the electric discharge device **(20)** to the air purification device **(10)** makes it possible to stabilize also the performance of the air purification device **(10)**. In addition, the using of a plurality of needle-shaped electrodes **(21a)** as the discharge electrode **(21)** provides an expanded discharge area, and it becomes possible to provide improvement in treatment performance by increasing the generation amount of active species. In this case, the discharge electrode **(21)** may be formed as follows. For example, a thin plate of stainless steel is formed into a shape having a plurality of needle parts **(21a)** each extending radially from a center part **(21b)** (see Figure **8(A)****).** The stainless steel thin plate thus formed is bent to obtain a member as shown in Figure **8(B)** and this member serves as the discharge electrode **(21)**.

Furthermore, as the electric power supply **(23)** used for the streamer discharge part **(20)**, a dc high voltage electric power supply or a pulse high voltage electric power supply may be used. In addition, in each of the first and third embodiments, the discharge electrode **(21)** is a positive pole. Conversely to this arrangement, the discharge electrode **(21)** may be a negative pole.

In addition, in the first embodiment, the ionization part **(30)** is provided in the air purification device **(10)** with a view to enhancing the effect of dust collection. However, it is not always necessary to provide the ionization part **(30).**

Furthermore, in each of the first to third embodiments, the liquid droplet ejector **(24)** is used and the counter electrode **(22)** is composed of liquid droplets. However, the counter electrode **(22)** does not always have to be in the form of liquid droplets. For example, as a substitute for the liquid droplet ejector **(24)**, it is possible to use a granular particulate ejector capable of ejecting minute granular particulates wherein such granular particulates ejected to the discharge electrode **(21)** from the granular particulate ejector serve as the counter electrode **(22)**. In addition, in this case, the "granular particulate" may be in the form a "powder particulate". The granular particulate ejector may employ a configuration capable of ejecting granular particulates or powder substances by means of pressure or wind power.

### INDUSTRIAL APPLICABILITY

As has been described above, the present description finds industrial utility in the field of electric discharge devices (such as the electric discharge device **(20)**) for the generation of a low temperature plasma by means of electric discharge, and air purification devices (such as the air purification device **(10)**) for the purification of air by making utilization of a low temperature plasma generated by such an electric discharge device.

## Claims

1. An electric discharge device, which device includes a discharge electrode (21), counter means (22), and an electric power supply (23) for providing an electric potential difference between the discharge electrode (21) and the counter means (22), for the generation of an electric discharge towards the counter means (22) from the discharge electrode (21),
**characterized by**
a liquid droplet ejector (24) oriented to eject liquid droplets towards the discharge electrode (21) which ejected liquid droplets form the counter means (22),
a streamer discharge part (20) including
the discharge electrode (21),
the liquid droplet ejector (24) and
a power supply (23) providing an electric potential difference between the discharge electrode (21) and the liquid droplet ejector (24) which streamer discharge part (20) generates a streamer discharge between discharge electrode (21) and the liquid droplets.

2. The electric discharge device (20) of claim 1, wherein the discharge electrode (21) is formed by a plurality of needle-shaped electrodes (21a).

3. The electric discharge device (20) of claim 1 or claim 2, wherein the tip of the discharge electrode (21) is oriented to the liquid droplet ejector (24).

4. The electric discharge device (20) of claim 3, wherein the shape of ejection pattern of the liquid droplets from the liquid droplet ejector (24) is a hollow circular cone.

5. The electric discharge device (20) of claim 3, wherein the shape of ejection pattern of the liquid droplets from the liquid droplet ejector (24) is a solid circular cone.

6. The electric discharge device (20) of claim 1 or 2, wherein the tip of the discharge electrode (21) is substantially oriented from sideways to the direction of flow of the liquid droplets ejected linearly from the liquid droplet ejector (24).

7. The electric discharge device (20) of any one of claims 1-6, wherein there is provided a liquid droplet recovery container (42) for the recovery of the liquid droplets ejected from the liquid droplet ejector (24).

8. The electric discharge device (20) of claim 7, wherein there is provided a liquid droplet circulating mechanism for the return of the liquid droplets recovered in the liquid droplet recovery container (42) towards the liquid droplet ejector (24).

9. An air purification device for the treatment of air to be treated and containing an odorous component, wherein the air purification device includes an electric discharge device (20) for the decomposition of the odorous component;
**characterized in that**
the electric discharge device (20) is formed by any one of the electric discharge devices (20) as set forth in claims 1 through 8.

10. The air purification device of claim 9, wherein a liquid droplet recovery unit is disposed downstream, relative to the direction of flow of the air to be treated, of the electric discharge device (20).

11. The air purification device of claim 9, wherein the liquid droplet ejector (24) is configured such that the liquid droplets are ejected in a direction opposite to the direction of flow of the air to be treated.

12. The air purification device of claim 9, wherein dust collecting means is disposed upstream, relative to the direction of flow of the air to be treated, of the electric discharge device (20).

13. The air purification device of claim 9, wherein a catalytic treatment part is disposed downstream, relative to the direction of flow of the air to be treated, of the electric discharge device (20).

14. The air purification device of claim 9, wherein an adsorptive treatment part is disposed downstream, relative to the direction of flow of the air to be treated, of the electric discharge device (20).

15. The air purification device of claim 9, wherein there is provided temperature controlling means for controlling the temperature of the air to be treated.

## Patentansprüche

1. Elektrische Entladungsvorrichtung, wobei die Vorrichtung eine Entladungselektrode (21), ein Aufnahmemittel (22) und eine elektrische Stromversorgung (23) zum Bereitstellen einer elektrischen Potenzialdifferenz zwischen der Entladungselektrode (21) und dem Aufnahmemittel (22) zur Erzeugung einer elektrischen Entladung in Richtung der Aufnahmemittel (22) von der Entladungselektrode (21) umfasst,
gekennzeichnet durch
eine Flüssigkeitströpfchenausstoßvorrichtung (24), ausgerichtet zum Ausstoßen von Flüssigkeitströpfchen in Richtung der Entladungselektrode (21), welche Flüssigkeitströpfchen von dem Aufnahmemittel (22) ausstieß,
eine Steamerentladungskomponente (20), umfassend
die Entladungselektrode (21),
die Flüssigkeitströpfchenausstoßvorrichtung (24) und
eine Stromversorgung (23), welche eine elektrische Potenzialdifferenz zwischen der Entladungselektrode (21) und der Flüssigkeitströpfchenaussstoßvorrichtung (24) bereitstellt, wobei Streamerentladungskomponente (20) eine Streamerentladung zwischen der Entladungselektrode (21) und den Flüssigkeitströpfchen erzeugt.

2. Elektrische Entladungsvorrichtung (20) nach Anspruch 1, wobei die Entladungselektrode (21) durch eine Vielzahl von nadelförmigen Elektroden (21a) gebildet wird.

3. Elektrische Entladungsvorrichtung (20) nach Anspruch 1 oder Anspruch 2, wobei die Spitze der Entladungselektrode (21) in Richtung der Flüssigkeitströpfchenausstoßvorrichtung (24) orientiert ist.

4. Elektrische Entladungsvorrichtung (20) nach Anspruch 3, wobei die Form des Ausstoßmusters der Flüssigkeitströpfchen von der Flüssigkeitströpfchenausstoßvorrichtung (24) ein hohler kreisförmiger Kegel ist.

5. Elektrische Entladungsvorrichtung (20) nach Anspruch 3, wobei die Form des Ausstoßmusters der Flüssigkeitströpfchen von der Flüssigkeitströpfchenausstoßvorrichtung (24) ein massiver kreisförmiger Kegel ist.

6. Elektrische Entladungsvorrichtung (20) nach Anspruch 1 oder 2, wobei die Spitze der Entladungselektrode (21) im Wesentlichen von der Seite in die Flussrichtung der Flüssigkeitströpfchen, die linear von der Flüssigkeitströpfchenausstoßvorrichtung (24) ausgestoßen werden, orientiert ist.

7. Elektrische Entladungsvorrichtung (20) nach einem der Ansprüche 1-6, wobei ein Flüssigkeitströpfchensammelbehälter (42) zum Sammeln der von der Flüssigkeitströpfchenausstoßvorichtung (24) ausgestoßenen Flüssigkeitströpfchen bereitgestellt wird.

8. Elektrische Entladungsvorrichtung (20) nach Anspruch 7, wobei ein Flüssigkeitströpfchenzyrkulationsmechanismus zum Zurückführen der in dem Flüssigkeitströpfchensammelbehälter (42) gesammelten Flüssigkeitströpfchen zu der Flüssigkeitströfchenausstoßvorrichtung (24) bereitgestellt wird.

9. Luftreinigungsvorrichtung zur Behandlung von zu behandelnder Luft, welche eine Riechstoff bildende Komponente umfasst, wobei die Luftreinigungsvorrichtung eine elektrische Entladungsvorrichtung (20) zum Zersetzen der Riechstoff bildenden Komponente umfasst;
**dadurch gekennzeichnet, dass**
die Entladungsvorrichtung (20) von irgendeiner der elektrischen Entladungsvorrichtungen nach den Ansprüchen 1 bis 8 geformt wird.

10. Luftreinigungsvorrichtung nach Anspruch 9, wobei eine Flüssigkeitströpfchensammeleinheit relativ zur Fließrichtung der zu behandelnden Luft stromabwärts von der elektrischen Entladungsvorrichtung (20) eingerichtet ist.

11. Luftreinigungsvorrichtung nach Anspruch 9, wobei die Flüssigkeitströpfchenausstoßvorrichtung (24) dazu eingerichtet ist, dass die Flüssigkeitsströpfchen in einer Richtung entgegen der Richtung des Flusses der zu behandelnden Luft ausgestoßen werden.

12. Luftreinigungsvorrichtung nach Anspruch 9, wobei ein Staubsammelmittel relativ zur Fließrichtung der zu behandelnden Luft stromaufwärts von der elektrischen Entladungsvorrichtung (20) angeordnet ist.

13. Luftreinigungsvorrichtung nach Anspruch 9, wobei ein katalytischer Behandlungsteil relativ zur Fließrichtung der zu behandelnden Luft stromabwärts von der elektrischen Entladungsvorrichtung (20) angeordnet ist.

14. Luftreinigungsvorrichtung nach Anspruch 9, wobei ein adsorbierender Behandlungsteil relativ zur Fließrichtung der zu behandelnden Luft stromabwärts von der elektrischen Entladungsvorrichtung (20) angeordnet ist.

15. Luftreinigungsvorrichtung nach Anspruch 9, wobei ein Temperatursteuermittel zum Steuern der zu behandelnden Luft bereitgestellt wird.

## Revendications

1. Dispositif à décharge électrique, lequel dispositif comprend une électrode de décharge (21), un moyen compteur (22), et une alimentation électrique (23) pour fournir une différence de potentiel électrique entre l'électrode de décharge (21) et le moyen compteur (22), pour la production d'une décharge électrique vers le moyen compteur (22) depuis l'électrode de décharge (21),
**caractérisé par**
un éjecteur de gouttelettes de liquide (24) orienté de façon à éjecter des gouttelettes de liquide vers l'électrode de décharge (21), lesquelles gouttelettes de liquide forment le moyen compteur (22),
une partie de décharge filamentaire (20) comprenant
l'électrode de décharge (21),
l'éjecteur de gouttelettes de liquide (24) et
une alimentation électrique (23) fournissant une différence de potentiel électrique entre l'électrode de décharge (21) et l'éjecteur de gouttelettes de liquide (24), la partie de décharge filamentaire (20) produisant une décharge filamentaire entre l'électrode de décharge (21) et les gouttelettes de liquide

2. Dispositif à décharge électrique (20) selon la revendication 1, dans lequel l'électrode de décharge (21) est formée par une pluralité d'électrodes en forme d'aiguilles (21a).

3. Dispositif à décharge électrique (20) selon la revendication 1 ou la revendication 2, dans lequel la pointe de l'électrode de décharge (21) est orientée vers l'éjecteur de gouttelettes de liquide (24).

4. Dispositif à décharge électrique (20) selon la revendication 3, dans lequel la forme du motif d'éjection des gouttelettes de liquide provenant de l'éjecteur de gouttelettes de liquide (24) est un cône circulaire creux.

5. Dispositif à décharge électrique (20) selon la revendication 3, dans lequel la forme du motif d'éjection des gouttelettes de liquide provenant de l'éjecteur de gouttelettes de liquide (24) est un cône circulaire plein.

6. Dispositif à décharge électrique (20) selon la revendication 1 ou 2, dans lequel la pointe de l'électrode de décharge (21) est orientée essentiellement depuis le côté vers le sens d'écoulement des gouttelettes de liquide éjectées linéairement depuis l'éjecteur de gouttelettes de liquide (24)

7. Dispositif à décharge électrique (20) selon l'une quelconque des revendications 1 à 6, dans lequel il est prévu un récipient de récupération des gouttelettes de liquide (42) pour la récupération des gouttelettes de liquide éjectées de l'éjecteur de gouttelettes de liquide (24).

8. Dispositif à décharge électrique (20) selon la revendication 7, dans lequel il est prévu un mécanisme de circulation des gouttelettes de liquide pour le retour des gouttelettes de liquide récupérées dans le récipient de récupération des gouttelettes de liquide (42) vers l'éjecteur de gouttelettes de liquide (24).

9. Dispositif d'épuration de l'air pour le traitement d'air à traiter et contenant un élément malodorant, ce dispositif d'épuration de l'air comprenant un dispositif à décharge électrique (20) pour la décomposition de l'élément malodorant ;
**caractérisé en ce que**
le dispositif à décharge électrique (20) est formé par n'importe lequel des dispositifs à décharge électrique (20) selon les revendications 1 à 8.

10. Dispositif d'épuration de l'air selon la revendication 9, dans lequel une unité de récupération de gouttelettes de liquide est disposée en aval du dispositif à décharge électrique (20), par rapport au sens d'écoulement de l'air à traiter.

11. Dispositif d'épuration de l'air selon la revendication 9, dans lequel l'éjecteur de gouttelettes de liquide (24) est configuré de manière à ce que les gouttelettes de liquide soient éjectées dans un sens opposé au sens d'écoulement de l'air à traiter.

12. Dispositif d'épuration de l'air selon la revendication 9, dans lequel un moyen de dépoussiérage est disposé en amont du dispositif à décharge électrique (20), par rapport au sens d'écoulement de l'air à traiter.

13. Dispositif d'épuration de l'air selon la revendication 9, dans lequel une partie de traitement catalytique est disposée en aval du dispositif à décharge électrique (20), par rapport au sens d'écoulement de l'air à traiter.

14. Dispositif d'épuration de l'air selon la revendication 9, dans lequel une partie de traitement adsorptif est disposée en aval du dispositif à décharge électrique (20), par rapport au sens d'écoulement de l'air à traiter.

15. Dispositif d'épuration de l'air selon la revendication 9, dans lequel il est prévu un moyen de régulation de la température pour réguler la température de l'air à traiter.
